# EUROPÄISCHE PATENTSCHRIFT

(11) **EP 0 952 453 B1**
(45) Veröffentlichungstag und Bekanntmachung des Hinweises auf die Patenterteilung: **23.10.2002**
(21) Anmeldenummer: 99107620.9
(22) Anmeldetag: 16.04.1999
(51) Int. Cl.: G01N 33/84

(54) **Verfahren und Reagenz zur störungsfreien Bestimmung von Eisen**
Process and reagent for the determination of iron, free of interferences
Procédé et agent pour la détermination de fer libre de perturbations

(30) Priorität: 22.04.1998 DE 19817963
(43) Veröffentlichungstag der Anmeldung: 27.10.1999
(73) Patentinhaber: Roche Diagnostics GmbH, 68298 Mannheim (DE)
(72) Erfinder: Treiber, Wolfgang, 82362 Weilheim (DE)

(56) Entgegenhaltungen:
- EP-A1- 0 306 859
- EP-A2- 0 343 592

## Beschreibung

Die Erfindung betrifft ein Verfahren zur störungsfreien Bestimmung von Eisen in biologischen Proben, insbesondere Serum unter Freisetzung des gebundenen Eisen, Reduktion des freigesetzten Eisens zu Fe²⁺, Zugabe einer Farbreagenzlösung und photometrischen Messung des gebildeten Farbkomplexes sowie eine Reagenzienkombination, die insbesondere in Gegenwart höherer Mengen EDTA die störungsfreie Bestimmung von Eisen geeignet ist.

Eisenstoffwechselstörungen, insbesondere Eisenmangel und Eisenresorptionsstörungen sind vor allem in der weiblichen Bevölkerung weit verbreitet. Der Nachweis von Eisen in Körperflüssigkeiten, insbesondere im Serum gehört daher zu den Standardbestimmungen in der medizinischen Analytik. Eisen wird mit der Nahrung zugeführt und über die Schleimhaut des Darmes aufgenommen. An Transferrin in 3-wertigem Zustand gebunden wird es dann zum Knochenmark transportiert, wo es hauptsächlich in Hämoglobin eingebaut wird. Wird zu wenig Eisen aufgenommen, so kommt es zu anämischen Erscheinungen.

Die Bestimmung des Eisens im Serum gehört zu den am häufigsten durchgeführten Spurenelementanalysen in der klinischen Diagnostik. Hinsichtlich der Freisetzung des Eisens aus Transferrin sind prinzipiell zwei Testvarianten für die Bestimmung bekannt. Zum einen kann die Eisenablösung durch den Zusatz von Detergenziengemischen (z.B. EP 0 130 537), Guanidiniumchlorid (z.B. EP 0 343 592) oder beispielsweise harnstoffhaltige Denaturierungsmittel (z.B. DE 44 01 754) bei schwach saurem pH-Wert erfolgen. Zum anderen ist Eisen aus Transferrin im stark sauren Medium, insbesondere bei einem pH-Wert von ca. 2.5 oder niedriger ablösbar (Clin. Chem. vol. 26 (1980), 327-331).

Von Nachteil bei den genannten Testprinzipien ist jedoch, daß diese durch die Gegenwart von EDTA. welches häufig in biologischen Proben. beispielsweise durch entsprechende Vorbehandlungen, enthalten ist, gestört werden. Denn EDTA ist befähigt mit Eisen Komplexe zu bilden und das so gebundene Eisen steht daher nicht zur Bildung eines Farbkomplexes zur Verfügung. Dies kann insbesondere bei EDTA-Plasma als Probenmaterial zu falsch negativen Eisenwerten führen. Darüber hinaus besteht die Gefahr, daß bei entsprechenden Bestimmungen auf Analysenautomaten durch nicht vermeidbare Pipettier- oder Rührvorrichtungen oder durch ungenügend gereinigte Reaktionsgefäße EDTA aus anderen Testflüssigkeiten eingeschleppt wird, wodurch es zu einem verminderten Farbsignal und damit zu einer verminderten Wiederfindung des Eisens kommt.

Um diesen Nachteil auszuräumen, werden bei der Testvariante, die die Freisetzung des Eisens durch ein geeignetes Denaturierungsmittel erreicht, Zink (II)-Salze zugesetzt. Das eingeschleppte EDTA wird durch Zn²⁺-Ionen maskiert und eine entsprechende Störung durch Reaktionen von EDTA mit Fe²⁺-Ionen vermieden.

Testvarianten, bei denen die Eisen-Ablösung im stark sauren Medium erfolgt, werden dagegen durch den Zusatz von Zink(II)-Salzen nicht von durch EDTA bewirkten Störungen befreit. Weiterhin hat sich erwiesen, daß in Gegenwart hoher Salzkonzentrationen (ca. 4 mol/l) und/oder hoher Detergenzkonzentrationen (ca. 7%), ein "Herauskriechen" der Reagenzlösung aus den Reagenzflaschen zu beobachten ist und damit beispielsweise zur Zerstörung von Barcode-Etiketten und zur Korrision von Metallteilen an Analysenautomaten führt.

Demzufolge liegt der vorliegenden Erfindung die Aufgabe zugrunde, ein Verfahren bzw. Reagenz zur Bestimmung von Eisen in biologischem Probenmaterial zur Verfügung zu stellen, welches für die Eisen-Freisetzung bei stark sauren pH-Werten und/oder in Lösungen mit hohen Salz- bzw. Detergenzkonzentrationen geeignet ist, wobei in Gegenwart von EDTA keine essentiellen Störungen auftreten bzw. eine dadurch verursachte erniedrigte Wiederfindung von Eisen vermieden wird.

Die Aufgabe wird gelöst durch ein Verfahren zur Bestimmung von Eisen in einer biologischen Probe unter Freisetzung des gebundenen Eisens, Reduktion des freigesetzten Eisens zu Fe²⁺, Zugabe einer Farbreagenzlösung und photometrische Messung des gebildeten Farbkomplexes, wobei die Probe mit einem wasserlöslichen Salz bestehend aus Metallkationen der III. Haupt- und Nebengruppe (III a+b) des Periodensystems der Elemente, wie beispielsweise Scandium, Indium, Lanthan oder Gallium, in Kontakt gebracht wird. Bevorzugt sind erfindungsgemäß Indiumund/oder Scandiumsalze. Das Gegenion der erfindungsgemäß zuzusetzenden Salze ist unkritisch, solange wasserlösliche Salze resultieren. Insbesondere haben sich hier zwei- oder dreiwertige Kationen sowie folgende Säurereste als geeignet erwiesen: Halogenide, Phosphate, Nitrate, Sulfite und Sulfate, wobei Chloride und Sulfate bevorzugt sind.

Die Konzentration der erfindungsgemäßen Salze der III. Haupt- bzw. Nebengruppe beträgt in der Reagenzlösung (Endkonzentration im Test) mindestens etwa 0,1 mmol/l, bevorzugt ist ein Bereich zwischen etwa 0,1 und 50 mmol/l, besonders bevorzugt eine Konzentration zwischen etwa 1 und 20 mmol/l.

Die Freisetzung des gebundenen Eisens kann bei dem erfindungsgemäßen Verfahren prinzipiell gemäß dem Fachmann bekannter Maßnahmen, wie beispielsweise durch den Zusatz geeigneter Detergenzienmischungen (z.B. EP 0 130 537, DE 27 24 757 oder DE 37 29 502) oder eines Denaturierungsmittels wie Guanidiniumchlorid (z.B. EP 0 343 592), bei pH-Werten von in der Regel zwischen 3 und 6 oder - ohne Zusatz einer Eisen freisetzenden Substanz - bei pH-Werten im stark Sauren erfolgen. Bei der Eisenablösung im stark sauren Medium hat sich erfindungsgemäß ein pH-Wert zwischen 0 und 3 als vorteilhaft erwiesen.

Zur Durchführung des erfindungsgemäßen Verfahrens wird die Probe bzw. das Reagenzgemisch bevorzugt in einem schwach und stark sauren Bereich, besonders bevorzugt im Bereich zwischen pH 0 und pH 6 gehalten. Als Puffersubstanzen sind Verbindungen geeignet, die einen pK-Wert von 2,5 bis 6 aufweisen und Eisen nicht oder nur schwach komplexieren. Geeignet sind beispielsweise Acetat-, Citrat-, Phosphat- oder Succinatpuffer. Eine weitere bevorzugte Variante besteht darin, zur Freisetzung des Eisens zunächst einen sauren pH-Wert einzustellen, besonders bevorzugt pH 3 bis pH 6, und danach auf einen pH-Wert umzupuffern, der mit dem zum Nachweis des Eisens gewählten Farbsystem eine optimale Farbausbeute ergibt. Die hierzu geeigneten pH-Werte sind dem Fachmann an sich bekannt, beispielsweise aus Fielding, Methods in Hematology 1 (Iron), 15-49 (1980). Besonders bevorzugt für das erfindungsgemäße Verfahren ist, wenn die Freisetzung des gebundenen Eisen im stark sauren Medium, bevorzugt zwischen pH 0 und 3, erfolgt und eine EDTA-komplexierende Substanz zugesetzt wird. Als EDTA-komplexierende Substanz kommen insbesondere Salze bestehend aus einem zwei- oder dreiwertigen Kation der Gruppen des Periodensystems III a, III b, IV a, VIII und/oder der Lanthaniden, wie beispielsweise Scandium, Indium, Zirconium, Nickel, Ytterbium und Lutetium in Betracht, wobei Scandium und/oder Indium bevorzugt sind.

Bevorzugt wird als Puffersubstanz Acetat- oder Citratpuffer eingesetzt. Der Puffer wird bevorzugt mit einer Konzentration von 20 bis 500 mmol/l, insbesondere in einem Bereich von 50 bis 250 mmol/l, verwendet. Ganz besonders bevorzugt ist, wenn die Pufferlösung ca. 200 mmol/l Citronensäure enthält. Die Puffersubstanz kann in der ersten Lösung des erfindungsgemäßen Eisentests, die zudem ein Denaturierungsmittel und gegebenenfalls Fettalkoholpolyglykolether enthalten kann, enthalten sein. Zusätzlich können auch die weiteren Lösungen oder die weitere Lösung des erfindungsgemäßen Eisentests gepuffert sein sowie weitere Hilfsstoffe zur Stabilisierung und/oder zur Eliminierung bzw. Reduzierung nicht gewünschter Reaktionen zugesetzt werden. So können beispielsweise durch den Zusatz von Fettalkoholpolyglykolether bzw. Alkylpolyethylenglykolether Störungen durch lipämische biologische Proben vermieden werden (DE 44 01 754). Eine weitere vorteilhafte Ausführungsform des erfindungsgemäßen Verfahrens ist, wenn dem Reagenz Thioharnstoff, besonders bevorzugt zwischen 10 und 200 mM zugesetzt wird. Dadurch können insbesondere Kupfer-Interferenzen eliminiert werden.

Zur Bestimmung des Eisens wird in an sich bekannter Weise der Probelösung ein Reduktionsmittel, wie zum Beispiel Ascorbinsäure, Dithionit oder Hydroxylamin, zugesetzt, um das in 3-wertiger Form vorliegende freigesetzte Eisen in die 2-wertige Form zu reduzieren. Das Reduktionsmittel kann sowohl in der ersten Lösung zusammen mit dem gegebenenfalls verwendeten Denaturierungsmittel als auch in der zweiten Lösung, die weiterhin das für den Nachweis von Eisen geeignete Farbsystem enthält, zugesetzt werden. Bevorzugt wird das Reduktionsmittel jedoch der zweiten Lösung zugesetzt. Des weiteren ist bevorzugt, wenn das Reagenz zusätzlich eine das Reduktionsmittel stabilisierende Substanz, wie ein geeignetes Konservierungsmittel (z.B. Germall II), beinhaltet.

Farbsysteme zum Nachweis von Eisen sind zum Beispiel beschrieben in der EP 0 228 060, Clin. Biochem. 14 (1981), 311-315 und Clin. Chem. 23 (1979), 234-240. Besonders sind Komplexbildner vom Ferrointyp geeignet, die mit Eisen einen Farbstoff lieferten, der photometrisch ausgewertet werden kann. Als geeignete Substanzen sind Bathophenanthrolin und Ferrozin (3'(2'-Pyridyl)-5,6-diphenyl-1,2,4-triazin-sulfonsäure-dinatriumsalz oder 3'(2'-Pyridyl)-5,6-bis (4-phenylsulfonsäure)-1,2,4-triazin-mononatiumsalz) zu nennen. Die Farbstoffbildung erfolgt dabei proportional zum Eisengehalt der Probe und kann in an sich bekannter Weise photometrisch ausgewertet werden.

Mit der Erfindung ist es erstmals gelungen, Störungen von EDTA bei der photometrischen Bestimmung von Eisen, insbesondere auch in stark sauren Reagenzlösungen, zu vermeiden.

Ein weiterer Gegenstand der Erfindung ist daher die Verwendung von wasserlöslichen Indiumund/oder Scandiumsalzen zur Verhinderung von durch die Anwesenheit von EDTA verursachten Störungen bei der photometrischen Bestimmung von Eisen in einer biologischen Probe.

Die Überprüfung der Wirksamkeit des Reagenzes zur Vermeidung von EDTA-Störungen kann photometrisch bei einem Probe-/Reagenzverhältnis von 1:5 bis 1:25, bevorzugt von 1:10 bis 1:15 erfolgen. Dabei konnte eine Wiederfindung von Eisen mit lediglich ca. 10% Abweichung erreicht werden. Die Messung erfolgt üblicherweise bei einer Temperatur von ca. 37°C sowie einer Wellenlänge von ca. 570-580 nm und anhand einer Küvette mit einer Schichtdicke von 1 cm gegen die Reagenzlösung als Leerwert.

Ein weiterer Gegenstand der Erfindung ist eine Reagenzienkombination zur Bestimmung von Eisen in einer biologischen Probe, gekennzeichnet durch ein erstes Reagenz, welches zur Freisetzung von gebundenem Eisen befähigt ist und etwa 0,1 bis 50 mmol/l eines wasserlöslichen Salzes bestehend aus einem Metallkation der III. Haupt- und/oder Nebengruppe, bevorzugt eines Indium- und/oder Scandiumsalzes enthält und getrennt davon ein zweites Reagenz, enthaltend 0,5 bis 50 mmol/l Farbstoff, und zwar in Form wäßriger Lösungen oder zu deren Herstellung geeigneter Trockengemische vorliegen. Beide Reagenzien oder auch nur das erste Reagenz können zusätzlich 20 bis 500 mmol/l Puffersubstanz enthalten.

Insbesondere das erste Reagenz kann darüber hinaus weitere Hilfsstoffe enthalten, wie beispielsweise ein zur Vermeidung lipämischer Interferenzen geeignetes Detergenz, bevorzugt sind hierbei etwa 1% bis 10% (w/v) Alkylpolyethylenglykolether (z.B. Genapol X-80: Isotridecylpoly (ethylenglycolether)n, n=8), oder zur Eliminierung von durch Kupfer bedingte Interferenzen, wobei 10 bis 200 mM Thioharnstoff bevorzugt sind.

Das zweite Reagenz kann zusätzlich ein Reduktionsmittel, beispielsweise Ascorbinsäure, Dithionit oder Hydroxylamin, in einer Konzentration von 20 bis 200 mmol/l enthalten. Wahlweise kann das zweite Reagenz zudem eine das Reduktionsmittel stabilisierende Substanz bzw. Substanzmischung, wie beispielsweise die käuflich zu erwerbenden Substanzen Germall II, Germall 115, Oxaban A oder Oxaban E in einer Konzentration von 0,5 bis 6,0 g/l, bevorzugt von 1 bis 3 g/l enthalten. Außerdem besteht die Möglichkeit, das Reduktionsmittel erst kurz vor der Verwendung dem Flüssigreagenz zuzugeben.

Die beiden Reagenzien können in Form wäßriger Lösungen oder zu deren Herstellung geeigneter Trockengemische (Lyophilisate) vorliegen.

Die Reagenzienkombination zeichnet sich darüber hinaus durch eine überraschend hohe Stabilität aus. So hat sich erwiesen, daß das Reagenz nach Lagerung von 24 Stunden bei 42°C bzw. 3 Wochen bei 35°C (entspricht einem Belastungsmodell für 18 Monate Realtime bei 2-8°C) ohne jeglichen Qualitäts- bzw. Aktivitätsverlust für die störungsfreie Bestimmung von Eisen verwendet werden kann.

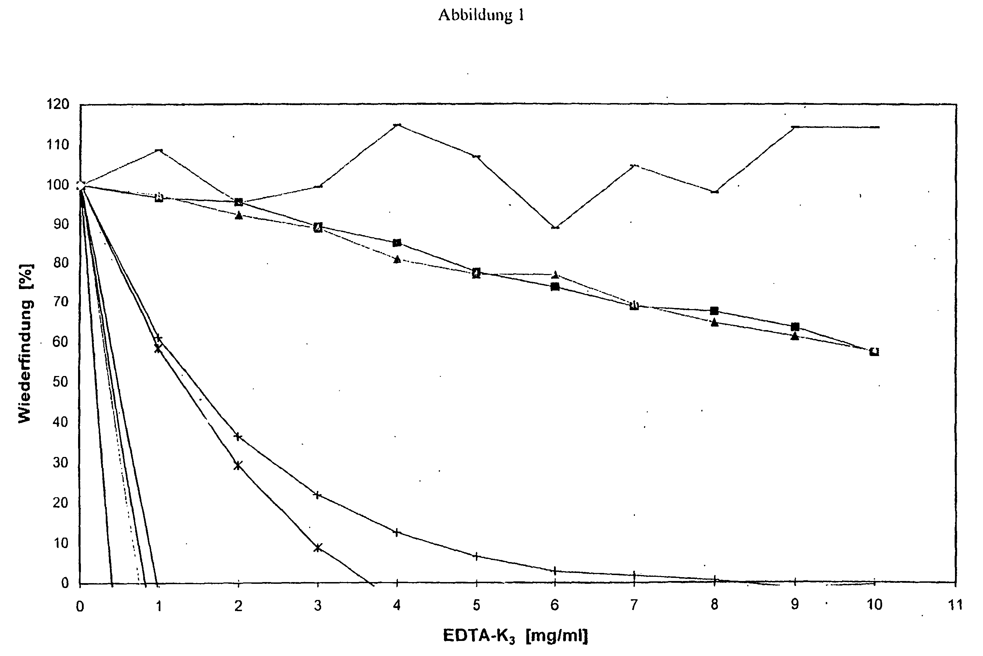

### Erläuterung der Abbildung:

### Abbildung 1:

Prozentuale Wiederfindung von Eisen in Gegenwart von 0 bis 10 mg/ml EDTA-Trikaliumsalz; Probe: Humanserumpool ca. 77 µg/dl Eisen; folgende Zusätze: 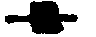 10 mM Indiumsulfat, 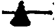 10 mM Scandiumsulfat, 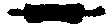 20 mM Nickelsulfat, 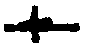 10 mM Ytterbiumsulfat, 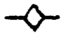 10 mM Zirkoniumsulfat, 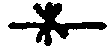 10 mM Lutetiumsulfat; übrige Kurven: ohne bzw. unwirksame Zusätze.

Die folgenden Beispiele erläutern die Erfindung näher.

### Beispiel 1

Zur Eisenbestimmung in Serum werden folgende Reagenzien eingesetzt:

| | | |
|---|---|---|
| Reagenz 1 | 200 mmol/l | Citronensäure |
| | 100 mmol/l | Thioharnstoff |
| | 7% (w/v) | Alkylpolyethylenglykolether |
| | 10mmol/l | Indium (III)-chlorid |
| | | |
| Reagenz 2 | 150 mmol/l | Natriumascorbat |
| | 6 mmol/l | 3'(2'-Pyridyl)-5,6-bis (4-Phenylsulfonsäure)-1,2,3-Triazin-mononatriumsalz |

Die Bestimmungen wurden an einem Hitachi 717-Analysenautomaten, Boehringer Mannheim GmbH, bei einer Meßtemperatur von 37°C durchgeführt. Die Messung erfolgte bei einer Wellenlänge von 570 nm gegen die Referenzwellenlänge 700 nm. Ausgewertet wurde die Differenz aus Meßwellenlänge und Referenzwellenlänge.

20 µl einer Serumprobe wurden mit 250 µl Reagenz 1 in eine Küvette pipettiert und nach Mischen bei 37°C inkubiert. Nach 5 Minuten wurde eine erste Extinktionsmessung durchgeführt. Anschließend wurden 50 µl Reagenz 2 zupipettiert, gemischt, weitere 5 Minuten inkubiert und dann eine zweite Extinktionsmessung durchgeführt. Die Auswertung erfolgte durch Differenzbildung der beiden Extinktionswerte.

In gleicher Weise wurde ein Leerwert mit 0,9%iger Kochsalzlösung als Probe und ein Kalibrator mit bekanntem Eisengehalt analysiert. Aus diesen beiden Proben wurde eine lineare Kalibrationskurve erstellt, an der die bekannten Proben anschließend abgelesen wurden.

### Ergebnisse:

Ein Humanserum wurde mit 3 mg/ml EDTA-K₃ aufgestockt (typische Konzentration eines EDTA-Plasmas) und als Probe eingesetzt. Als Vergleichswert wurde der unaufgestockte Humanserumpool gemessen und die Wiederfindung der aufgestockten Probe in Relation zur unaufgestockten Probe berechnet.

**Tabelle 1:**

| Zusatz zu Reagenz 1 | Wiederfindung |
|---|---|
| ohne Zusatz | -2,8% |
| 10mmol/l Indiumsulfat | 89,4% |
| 10 mmol/l Scandiumsulfat | 88,9% |
| 10 mmol/l Lutetiumsulfat | 8,8% |
| 10mmol/l Ytterbiumsulfat | 22,0% |
| 10 mmol/l Nickelsulfat | 79,8% |
| 10 mmol/l Zirkoniumsulfat | 16,7% |

## Patentansprüche

1. Verfahren zur Bestimmung von Eisen in einer biologischen Probe unter Freisetzung des gebundenen Eisens, Reduktion des freigesetzten Eisens zu Fe²⁺, Zugabe einer Farbreagenzlösung und photometrische Messung des gebildeten Farbkomplexes, **dadurch gekennzeichnet, daß** die Probe mit einem wasserlöslichen Salz bestehend aus einem Metallkation der III. Haupt- und/oder Nebengruppe (III a+b) des Periodensystems der Elemente versetzt wird.

2. Verfahren nach Anspruch 1, **dadurch gekennzeichnet, daß** die Konzentration des Metallsalzes zwischen 0,1 und 50 mmol/l beträgt.

3. Verfahren nach einem der Ansprüche 1 oder 2, **dadurch gekennzeichnet, daß** ein Indiumund/oder Scandiumsalz zugesetzt wird.

4. Verfahren nach einem der Ansprüche 1 bis 3, **dadurch gekennzeichnet, daß** weitere Hilfsstoffe zur Stabilisierung oder zur Vermeidung bzw. Reduzierung nicht gewünschter Reaktionen zugegen sind.

5. Verfahren nach einem der Ansprüche 1 bis 4, **dadurch gekennzeichnet, daß** der pH-Wert in der Testlösung zwischen 0 und 3 beträgt.

6. Verwendung von wasserlöslichen Salzen bestehend aus einem Metallkation der III. Hauptund/oder Nebengruppe zur Vermeidung von durch die Anwesenheit von EDTA verursachten Störungen bei der photometrischen Bestimmung von Eisen in einer Probe.

7. Reagenzienkombination zur Bestimmung von Eisen in einer biologischen Probe, **dadurch gekennzeichnet, daß** ein erstes Reagenz, welches zur Freisetzung des gebundenen Eisens befähigt ist und etwa 0,1 bis 50 mmol/l eines wasserlöslichen Salzes bestehend aus einem Metallkation der III. Haupt- und/oder Nebengruppe aufweist und getrennt davon ein zweites Reagenz, enthaltend etwa 0,5 bis 50 mmol/l Farbstoff, in Form wäßriger Lösungen oder zu deren Herstellung geeigneter Trockengemische.

8. Reagenzienkombination nach Anspruch 7, **dadurch gekennzeichnet, daß** im ersten Reagenz etwa 1% bis 10% (w/v) eines Alkylpolyethylenglykolethers enthalten sind.

9. Reagenzienkombination nach Anspruch 7 oder 8, **dadurch gekennzeichnet, daß** im zweiten Reagenz etwa 20 bis 200 mmol/l Reduktionsmittel und gegebenenfalls eine das Reduktionsmittel stabilisierende Substanz enthalten ist.

10. Reagenzienkombination nach einem der Ansprüche 7 bis 9, **dadurch gekennzeichnet, daß** das erste Reagenz oder das erste und das zweite Reagenz einen pH-Wert zwischen 0 und 3 aufweisen.

## Claims

1. Method for the determination of iron in a biological sample in which the bound iron is released, the released iron is reduced to Fe²⁺, a colour reagent solution is added and the colour complex that is formed is measured photometrically, wherein a water-soluble salt composed of a metal cation from the IIIrd main group and/or subgroup (III a + b) of the periodic system of the elements is added to the sample.

2. Method as claimed in claim 1, wherein the concentration of the metal salt is between 0.1 and 50 mmol/l.

3. Method as claimed in one of the claims 1 or 2, wherein an indium and/or, scandium salt is added.

4. Method as claimed in one of the claims 1 to 3, wherein other auxiliary substances are present for stabilization or to avoid or reduce undesired reactions.

5. Method as claimed in one of the claims 1 to 4, wherein the pH value in the test solution is between 0 and 3.

6. Use of water-soluble salts composed of a metal cation of the IIIrd main group and/or subgroup to avoid interference caused by the presence of EDTA in the photometric determination of iron in a sample.

7. Combination of reagents for the determination of iron in a biological sample, **characterized by** a first reagent which is able to release the bound iron and contains about 0.1 mmol/l to 50 mmol/l of a water-soluble salt composed of a metal cation of the IIIrd main group and/or subgroup and separate therefrom a second reagent containing about 0.5 to 50 mmol/l dye, in the form of aqueous solutions or dry mixtures suitable for their preparation.

8. Combination of reagents as claimed in claim 7, wherein the first reagent contains about 1 % to 10 % (w/v) of an alkylpolyethylene glycol ether.

9. Combination of reagents as claimed in claim 7 or 8, wherein the second reagent contains about 20 to 200 mmol/l reducing agent and optionally a substance which stabilizes the reducing agent.

10. Combination of reagents as claimed in one of the claims 7 to 9, wherein the first reagent or the first and the second reagent have a pH value between 0 and 3.

## Revendications

1. Procédé pour la détermination de la teneur en fer d'un échantillon biologique par libération du fer lié, réduction du fer libéré pour obtenir du Fe²⁺, addition d'une solution de réactif de coloration et mesure photométrique du complexe coloré obtenu, **caractérisé en ce qu'**on ajoute à l'échantillon un sel hydrosoluble constitué d'un cation métallique du groupe principal et/ou du sous-groupe III (Illa + b) du système périodique des éléments.

2. Procédé selon la revendication 1, **caractérisé en ce que** la concentration du sel métallique se situe entre 0,1 et 50 millimoles/l.

3. Procédé selon l'une quelconque des revendications 1 ou 2, **caractérisé en ce qu'**on ajoute un sel d'indium et/ou de scandium.

4. Procédé selon l'une quelconque des revendications 1 à 3, **caractérisé en ce qu'**on ajoute des adjuvants supplémentaires afin de stabiliser ou encore afin de supprimer, respectivement de réduire des réactions non désirées.

5. Procédé selon l'une quelconque des revendications 1 à 4, **caractérisé en ce que** la valeur de pH dans la solution d'essai se situe entre 0 et 3.

6. Utilisation de sels hydrosolubles constitués d'un cation métallique du groupe principal et/ou du sous-groupe III pour éviter des perturbations provoquées par la présence de EDTA lors de la détermination photométrique de la teneur en fer d'un échantillon.

7. Combinaison de réactifs pour la détermination de la teneur en fer d'un échantillon biologique, **caractérisée par** un premier réactif qui est capable de libérer le fer à l'état lié et qui présente, à concurrence de 0,1 à 50 millimoles/l, un sel hydrosoluble constitué d'un cation métallique du groupe principal et/ou du sous-groupe III et, à l'état séparé du premier cité, un deuxième réactif contenant un colorant à raison d'environ 0,5 à 50 millimoles/l, sous la forme de solutions aqueuses ou de mélanges secs appropriés pour la préparation desdites solutions.

8. Combinaison de réactifs selon la revendication 7, **caractérisée en ce que** le premier réactif contient un éther alkylique de polyéthylèneglycol à raison d'environ 1 % à 10 % (en poids/volume).

9. Combinaison de réactifs selon la revendication 7 ou 8, **caractérisée en ce que** le deuxième réactif contient un agent de réduction à raison d'environ 20 à 200 millimoles/l et le cas échéant une substance stabilisant l'agent de réduction.

10. Combinaison de réactifs selon l'une quelconque des revendications 7 à 9, **caractérisée en ce que** le premier réactif ou bien le premier et le deuxième réactif présentent une valeur de pH entre 0 et 3.
